# EUROPEAN PATENT APPLICATION

(11) **EP 4 596 103 A1**
(43) Date of publication of application: **06.08.2025**
(21) Application number: 23870885.3
(22) Date of filing: 27.09.2023
(51) Int. Cl.: B01J 27/232, C07C 2/84

(54) **LANTHANUM OXYCARBONATE CATALYST, PREPARATION METHOD THEREFOR AND USE THEREOF**

(30) Priority: 27.09.2022 CN 202211186899
(71) Applicant: China Petroleum & Chemical Corporation, Beijing 100728 (CN); Sinopec (Beijing) Research Institute of Chemical Industry Co., Ltd., Beijing 100013 (CN)
(72) Inventor: WU, Jiehua, Beijing 100013 (CN); XUE, Wei, Beijing 100013 (CN); MAN, Yi, Beijing 100013 (CN); FENG, Yingjie, Beijing 100013 (CN); LIU, Dongbing, Beijing 100013 (CN); ZHANG, Mingsen, Beijing 100013 (CN)
(74) Representative: Hoffmann Eitle
(86) International application number: PCT/CN2023/121840
(87) International publication number: WO 2024/067658

(57) **Abstract**

The invention relates to the technical field of lanthanum dioxide carbonate, and discloses a lanthanum dioxide carbonate catalyst, a preparation method and a use thereof. The lanthanum dioxide carbonate catalyst comprises hexagonal crystalline lanthanum dioxide carbonate and hexagonal crystalline lanthanum dioxide carbonate containing a doping element R. The method comprises: (1) adding an alkali solution to a solution of lanthanum source, and then performing a solid-liquid separation and a drying to obtain lanthanum hydroxide; (2) contacting a solution of a compound containing the doping element R with lanthanum hydroxide, and then performing a drying; (3) calcining the dried product of step (2) under a carbon-containing atmosphere to obtain a lanthanum dioxide carbonate catalyst. When the catalyst prepared by the invention is used in a methane oxidative coupling reaction, it has a high yield of C2 hydrocarbons.

## Description

### Technical Field

The invention relates to a lanthanum dioxide carbonate catalyst, and its preparation method and use.

### Background Art

Lanthanum dioxide carbonate is a new type of material, mainly used in fields of water treatment, optoelectronics, catalysis, etc. At present, the main methods for synthesizing lanthanum dioxide carbonate are precipitation method, lanthanum carbonate thermal decomposition method, and hydrothermal method. However, lanthanum dioxide carbonate is usually prepared by a two-steps method, first synthesizing La(OH)₃ precursor, and then calcining it at low temperature. At present, the improvement of lanthanum dioxide carbonate material is mainly achieved by changing the preparation method in order to obtain lanthanum dioxide carbonate with different morphologies or structures.

CN113797949A discloses a method for preparing lanthanum dioxide carbonate, which comprises synthesizing rod-shaped nanostructured lanthanum dioxide carbonate under ultrasonic conditions. The prepared lanthanum dioxide carbonate can efficiently carry out methane oxidative coupling reaction at a relatively low temperature, but the yield of C2 hydrocarbons of the lanthanum dioxide carbonate is relatively low.

There are few reports on element doping of lanthanum dioxide carbonate materials in the prior art; only a few documents have reported on impregnating lanthanum dioxide carbonate materials with solutions containing a doping element; however, there are no reports in the prior art on completing the element doping during the preparation process of lanthanum dioxide carbonate.

### Summary of The Invention

The present invention provides a lanthanum dioxide carbonate catalyst and its preparation method and use. The catalyst is particularly suitable for activating methane oxidative coupling and has a higher C2 hydrocarbons yield for example at 500-650°C.

In order to achieve the above-mentioned object, in one aspect, the present invention provides a lanthanum dioxide carbonate catalyst, which comprises hexagonal crystalline lanthanum dioxide carbonate and hexagonal crystalline lanthanum dioxide carbonate containing a doping element R; the total content of hexagonal crystalline lanthanum dioxide carbonate and hexagonal crystalline lanthanum dioxide carbonate containing a doping element R in the lanthanum dioxide carbonate catalyst is not less than 98wt% (preferably not less than 99wt% ), wherein the molar ratio of the lanthanum element to the doping element R is 1:0.01 to 1:0.3; and the doping element R is selected from at least one of the elements Mg, Ca, Sr, Ba, Fe and Zn.

Preferably, no characteristic peak of the doping element in the form of metal salts appears in the XRD spectrum of the catalyst of the present invention.

In another aspect, the present invention provides a method for preparing the lanthanum dioxide carbonate catalyst of the present invention, the method comprising:
(1) adding an alkali solution to a solution of lanthanum source, and then optionally performing an aging, performing a solid-liquid separation and a drying to obtain solid lanthanum hydroxide; wherein the drying comprises a first drying and a second drying, wherein the first drying conditions comprises: a temperature of 70-90°C and a time of 10-20 hours, and the second drying is carried out at a higher temperature and a shorter time than the first drying;
   determining the saturated water absorption amount of the lanthanum hydroxide after the second drying: under nitrogen protection, taking 1g of the lanthanum hydroxide after the second drying and placing it in a container, adding water to evenly mix it with the lanthanum hydroxide, until the lanthanum hydroxide has no obvious further water absorption, and recording the addition amount of water at this time as the saturated water absorption amount of the lanthanum hydroxide;
(2) optionally under a protective atmosphere, adding a solution of a compound containing the doping element R to the solid lanthanum hydroxide obtained in step (1) so that the solution in an amount less than or equal to the saturated water absorption amount of the lanthanum hydroxide is in contact with the lanthanum hydroxide, and then performing a drying; wherein the contacting method is selected from surface precipitation, atomic layer deposition and single atomic layer plating;
(3) calcining the dried product of step (2) in a carbon-containing (e.g. CO and/or CO₂) atmosphere to obtain a lanthanum dioxide carbonate catalyst.

In another aspect, the present invention provides a lanthanum dioxide carbonate catalyst prepared by the method described above.

In another aspect, the present invention provides a use of the above-mentioned lanthanum dioxide carbonate catalyst in the methane oxidative coupling reaction to produce C2 and higher hydrocarbons.

In another aspect, the present invention provides a method for preparing C2 and higher hydrocarbons from methane, the method comprising: in the presence of oxygen and under the conditions of methane oxidative coupling reaction, contacting methane with the lanthanum dioxide carbonate catalyst of the present invention for reaction; or,
preparing the lanthanum dioxide carbonate catalyst according to the method of the present invention, and then contacting methane with the obtained lanthanum dioxide carbonate catalyst in the presence of oxygen and under the conditions of methane oxidative coupling reaction.

The catalyst provided by the present invention comprises both hexagonal crystalline lanthanum dioxide carbonate and hexagonal crystalline lanthanum dioxide carbonate containing a doping element R, which can effectively promote the occurrence of methane oxidative coupling reaction and improve the yield of C2 hydrocarbons.

The present invention first prepares lanthanum hydroxide by adding alkali solution into a solution containing lanthanum element, and then contacting the solution containing the doping element R with lanthanum hydroxide to prepare a catalyst. The prepared catalyst comprises hexagonal crystalline lanthanum dioxide carbonate and hexagonal crystalline lanthanum dioxide carbonate containing a doping element R. When the catalyst prepared by the present invention is used for methane oxidative coupling reaction, a higher yield of C2 hydrocarbons is obtained.

### Brief Description of Figures

FIG.1 is an XRD spectrum of the lanthanum dioxide carbonate catalyst prepared in Example 1;
FIG.2 is a transmission scanning electron micrograph image of the lanthanum dioxide carbonate catalyst prepared in Example 1;
FIG.3 is an XRD spectrum of the lanthanum dioxide carbonate catalyst prepared in Comparative Example 4.

### Detailed Description of The Invention

The endpoints and any values of the ranges disclosed herein are not limited to the precise ranges or values, and these ranges or values should be understood to include values close to these ranges or values. For numerical ranges, the endpoint values of each range, the endpoint values and the individual point values of each range, and the individual point values can be combined with each other to obtain one or more new numerical ranges, which should be considered as specifically disclosed herein.

Where not expressly indicated, all references herein to percentages, parts, ratios, and the like are made on a weight basis, except where not in accordance with the conventional understanding of those skilled in the art when made on a weight basis.

In the description, unless otherwise specified, the terms "include(s)", ''comprise(s)", "contain(s)", "have(has)" and similar expressions represent open-ended situations, but should also be understood to explicitly disclose close-ended situations at the same time. For example, "include" means that other elements not listed may also be included, but it also explicitly discloses that only the listed elements are included. In addition, as used herein, "include/comprise" is interpreted as explicitly stating the presence of the mentioned features, integers, steps or components, but does not exclude the presence or addition of one or more other features, integers, steps, components or groups thereof. In addition, the term "comprise" is intended to include embodiments covered by the terms "essentially consist of..." and "consist of...". Similarly, the term "essentially consist of..." is intended to include embodiments covered by the term "consist of..."

In one aspect, the present invention provides a lanthanum dioxide carbonate catalyst, which comprises hexagonal crystalline lanthanum dioxide carbonate and hexagonal crystalline lanthanum dioxide carbonate containing a doping element R; the total content of the hexagonal crystalline lanthanum dioxide carbonate and the hexagonal crystalline lanthanum dioxide carbonate containing a doping element R in the lanthanum dioxide carbonate catalyst is not less than 98wt% (preferably not less than 99wt%), wherein the molar ratio of the lanthanum element to the doping element R is 1:0.01 to 1:0.3; and the doping element R is selected from at least one of the elements Mg, Ca, Sr, Ba, Fe and Zn.

Without being limited to any known theory, it is believed that the present invention benefits from the factors including but not limited to the following: performing an appropriate control of the molar ratio of the lanthanum element to the doping element R, and in particular the specific preparation method makes the lanthanum dioxide carbonate and the lanthanum dioxide carbonate containing a doping element R in the obtained lanthanum dioxide carbonate catalyst mostly (not less than 98wt%, preferably not less than 99wt%) exist in the form of hexagonal crystalline phase, and ensures a satisfactory catalyst structure, including the content of the doping element and its distribution, which is beneficial to the activity of the catalyst, especially when it is used to activate methane oxidative coupling, a higher methane conversion rate, as well as a higher selectivity and yield of C2 hydrocarbons are obtained.

According to the present invention, the total content of hexagonal crystalline lanthanum dioxide carbonate and hexagonal crystalline lanthanum dioxide carbonate containing a doping element R in the lanthanum dioxide carbonate catalyst is not less than 98wt%, preferably not less than 99wt%, and may even be not less than 99.5wt%, or close to 100wt%; this means that although the lanthanum dioxide carbonate has been treated in several steps in the present invention, the crystalline phase in the obtained lanthanum dioxide carbonate catalyst is absolutely dominated by the hexagonal crystalline phase, which ensures the high purity of the hexagonal crystalline phase relative to other crystalline phases.

For the purpose of the present invention, the content of hexagonal crystalline phase, such as the total content of the hexagonal crystalline phase lanthanum dioxide carbonate and hexagonal crystalline phase lanthanum dioxide carbonate containing a doping element R, is determined by XRD spectrum. Specifically, for example, the bulk crystal structure information is measured by XRD spectrum, and the area of diffraction peaks of the hexagonal crystalline lanthanum dioxide carbonate and the crystal structure containing a doping element and the area of all peaks are used to calculate the ratio thereof, which is the weight percentage of the hexagonal crystalline lanthanum dioxide carbonate and the hexagonal crystalline lanthanum dioxide carbonate containing a doping element in the product.

According to the present invention, preferably, the doping element R can be selected from at least one of the elements of Groups IIA, VIII, IB and IIB, more preferably at least one of the elements Mg, Ca, Sr, Ba, Fe and Zn, and further preferably Sr and/or Ba. For other application purposes, there are some reports in the prior art that Ni is added to lanthanum dioxide carbonate; however, without being limited to any known theory, it is believed that, especially when used for activating methane oxidative coupling, the doping element R selected from Mg, Ca, Sr, Ba, Fe and Zn is particularly conducive to higher methane conversion, and higher selectivity and yield of C2 hydrocarbons, especially higher yield of C2 hydrocarbons.

According to the present invention, preferably, the molar ratio of the lanthanum element to the doping element R in the lanthanum dioxide carbonate catalyst is 1:0.03 to 1:0.2, preferably from 1:0.04 to 1:0.15. The molar ratio of the lanthanum element to the doping element R in the lanthanum dioxide carbonate catalyst can be calculated based on the feed amounts of lanthanum element and the doping element R.

According to the present invention, preferably, the lanthanum dioxide carbonate catalyst has a fibrous nanostructure, the diameter of the lanthanum dioxide carbonate catalyst is 10nm-30nm, preferably 10nm-20nm, and the length-to-diameter ratio is 5-50: 1, more preferably 15-40: 1.

In the present invention, "particle size/diameter" and "length-to-diameter ratio" of the lanthanum dioxide carbonate catalyst are both average values, wherein the specific measuring method for the "average particle size/diameter" is: using a transmission electron microscope ruler, selecting 5-10 samples in the view window, measuring the diameter of each sample, and then calculating the average value. The specific measuring method for the "average length-to-diameter ratio" is: using a transmission electron microscope ruler, selecting 5-10 samples in the view window, measuring the diameter and length of each sample respectively, and then calculating the length-to-diameter ratio of each sample, and then calculating the average value.

According to the present invention, preferably, the lanthanum dioxide carbonate catalyst has a specific surface area of 35m²/g-90m²/g, preferably 45m²/g-85m²/g, and further preferably 50m²/g-60m²/g, a pore volume of 0.15cm³/g-0.5cm³/g, preferably 0.26cm³/g-0.4cm³/g, and an average pore diameter of 8 nm-16 nm, preferably 8 nm-15 nm, and further preferably 10 nm-13.5 nm.

In another aspect, the present invention provides a method for preparing the lanthanum dioxide carbonate catalyst of the present invention, the method comprising:
(1) adding an alkali solution to a solution of lanthanum source, and then optionally performing an aging, performing a solid-liquid separation and a drying to obtain solid lanthanum hydroxide; wherein the drying comprises a first drying and a second drying, the first drying conditions comprising: a temperature of 70-90°C and a time of 10-20 hours, and the second drying is carried out at a higher temperature and a shorter time than the first drying;
   determining the saturated water absorption amount of the lanthanum hydroxide after the second drying: under nitrogen protection, taking 1g of the lanthanum hydroxide after the second drying and placing it in a container, adding water to evenly mix it with the lanthanum hydroxide, until the lanthanum hydroxide has no obvious further water absorption, and recording the addition amount of water at this time as the saturated water absorption amount of the lanthanum hydroxide;
(2) optionally under a protective atmosphere, adding a solution of a compound containing the doping element R to the solid lanthanum hydroxide obtained in step (1) so that the solution is contacted with the lanthanum hydroxide, and then performing a drying, wherein the amount of the solution of a compound containing the doping element R in contact with the lanthanum hydroxide is less than or equal to (preferably equal to or substantially equal to) the saturated water absorption amount of the lanthanum hydroxide; wherein the contacting method is selected from surface precipitation, atomic layer deposition and single atomic layer plating;
(3) calcining the dried product of step (2) in a carbon-containing (such as CO and/or CO₂) atmosphere to obtain a lanthanum dioxide carbonate catalyst.

According to the present invention, the above-mentioned specific preparation method makes the lanthanum dioxide carbonate and lanthanum dioxide carbonate containing a doping element R in the obtained lanthanum dioxide carbonate catalyst mostly exist in the form of hexagonal crystalline phase, so that in the obtained lanthanum dioxide carbonate catalyst, the total content of hexagonal crystalline lanthanum dioxide carbonate and hexagonal crystalline lanthanum dioxide carbonate containing a doping element R is not less than 98wt%, preferably not less than 99wt%, and even can be not less than 99.5wt%, or close to 100wt%.

According to the present invention, in order to make the resulting lanthanum hydroxide more fibrous in morphology, preferably, the concentration of the alkali solution is 3wt%-25wt%.

According to the present invention, preferably, the alkali in the alkali solution is a compound of a Group IA metal.

According to the present invention, preferably, the addition rate of the alkali solution is 5 mL/min-150 mL/min per kilogram of the solution of lanthanum source.

According to the present invention, preferably, the concentration of the solution of lanthanum source is 0.01wt%-10wt%.

According to the present invention, preferably, the utilization amount of the alkali solution is such that the final pH value of the mixed system of the alkali solution and the solution of lanthanum source is 10-12.5.

According to the present invention, the lanthanum source can be any substance that can provide lanthanum element. Preferably, the lanthanum source is a water-soluble salt of lanthanum, more preferably at least one of lanthanum nitrate, lanthanum chloride and lanthanum acetate; further preferably lanthanum nitrate.

According to the present invention, when the aging is performed, the present invention has no particular limitation on the aging device, as long as the aging temperature and stirring conditions can be met, but based on the safety of the experiment and the comprehensive catalyst performance, the aging is performed under condensation reflux. Preferably, the aging conditions include: a temperature of 80-100°C and a time of 10-50h.

The present invention may further include: performing a solid-liquid separation of the aged product, and then washing it with deionized water until it is neutral. Wherein the solid-liquid separation method may be a conventional solid-liquid separation method in the art, such as performing the separation using a centrifuge.

According to the present invention, preferably, in step (1), the drying includes a first drying and a second drying, so as to obtain the solid lanthanum hydroxide.

According to the present invention, preferably, the first drying conditions include: a temperature of 70-90°C, and a time of 10-20 h.

According to the present invention, preferably, the second drying conditions include: a relative pressure of 10kPa-91kPa, preferably 20kPa-70kPa, a temperature of 120-160°C, and a time of 2-10 h; wherein the second drying is performed for a shorter time than the first drying.

Without being limited to any known theory, it is believed that separately carrying out the first drying and the second drying in the above step (1) and more accurately controlling the drying conditions (such as temperature and time) are particularly beneficial to the purpose of the present invention, such as controlling the crystalline phase.

According to the present invention, when the protective atmosphere is used, preferably, the protective atmosphere is provided by an inert gas and/or nitrogen.

In the present invention, the protective atmosphere can be provided by using Schlenk technology (double-row pipeline operation) or in a glove box with a protective atmosphere.

According to the present invention, preferably, it step (1), it further includes a process of measuring the saturated water absorption amount of lanthanum hydroxide. Then, a solution of a compound containing the doping element R is prepared according to the measured saturated water absorption amount of lanthanum hydroxide and the utilization amount of the doping element R.

According to the present invention, preferably, in step (2), according to the measured saturated water absorption amount of lanthanum hydroxide, a solution of a compound containing the doping element R in an amount less than or equal to (or substantially equal to) the saturated water absorption amount of lanthanum hydroxide is added to the solid lanthanum hydroxide obtained in step (1). For the purpose of the present invention, the addition amount of the solution of a compound containing the doping element R may preferably be "equal to" the saturated water absorption amount of lanthanum hydroxide; however, the mixing of the solution and the solid may cause a slight change in volume, and thus, the addition amount of the solution of a compound containing the doping element R calculated without considering the slight change in volume can be regarded as "substantially equal to" the saturated water absorption amount of the lanthanum hydroxide. Without being limited to any known theory, it is believed that adding the solution of a compound containing the doping element R according to the measured saturated water absorption amount of lanthanum hydroxide is particularly beneficial to the purpose of the present invention, such as controlling the amount of solution and the amount of doping element in the method, thereby being able to obtain a desired doping amount without causing a change in the crystalline phase.

According to the present invention, preferably, the concentration of the solution of a compound containing the doping element R is 0.01-0.2 g/mL.

According to the present invention, preferably, the molar ratio of the lanthanum element to the doping element R in lanthanum hydroxide is 1: 0.01-1, preferably 1: 0.03-0.5, and more preferably 1: 0.04-0.2.

According to the present invention, preferably, the compound containing the doping element R is selected from compounds of at least one element in Groups IIA, VIII, IB and IIB, more preferably compounds of at least one element Mg, Ca, Sr, Ba, Fe and Zn, further preferably compounds of Sr and/or Ba. The compound containing the doping element R may be at least one of a nitrate (such as magnesium nitrate, calcium nitrate, strontium nitrate, barium nitrate, ferric nitrate, zinc nitrate), a chloride (such as magnesium chloride, calcium chloride, strontium chloride, barium chloride, ferric chloride, zinc chloride) and an acetate (such as magnesium acetate, calcium acetate, strontium acetate, barium acetate, ferric acetate, zinc acetate) of the doping element R, preferably a nitrate.

According to the present invention, preferably, in step (2), the solution of a compound containing the doping element R is added to the solid lanthanum hydroxide obtained in step (1) by a specific contact method, rather than adding the solution of a compound containing the doping element R to the lanthanum hydroxide obtained in step (1). Without being limited to any known theory, it is believed that the specific addition method is conducive to the achievement of the object of the present invention, especially, for example, the distribution of doping element, and accordingly, the higher conversion rate and selectivity, and especially the yield brought by the obtained catalyst. Thus, for the purpose of the present invention, a suitable specific contact method can be selected from lattice doping, surface precipitation, atomic layer deposition and single atomic layer plating. An example of a specific contact method that can be mentioned is surface precipitation, which includes: under stirring conditions, adding dropwise the solution of a compound containing the doping element R to lanthanum hydroxide, and continuing to stir after the addition is completed. More preferably, the addition rate of the solution of a compound containing the doping element R is 0.1mL/min-10mL/min, preferably 0.1mL/min-8mL/min, per gram of the lanthanum hydroxide.

According to the present invention, preferably, the contact conditions include: a temperature of 15-50°C and a time of 2-5 h.

According to the present invention, in order to fully contact the solution of a compound containing the doping element R with lanthanum hydroxide, the contact is carried out in a vortex mixer.

According to the present invention, preferably, in step (2), the drying conditions include: a temperature of 60-100°C. and a time of 10-24 h.

According to the present invention, preferably, in step (3), the calcination conditions include: a temperature of 450-550°C and a time of 2-8h. According to the present invention, the carbon-containing atmosphere can be any atmosphere that can provide carbon for the purpose of the present invention and will not be detrimental to obtaining the desired lanthanum dioxide carbonate catalyst, especially such as an atmosphere containing CO and/or CO₂. Preferably, the carbon-containing atmosphere (such as CO and/or CO₂) can be an air atmosphere; accordingly, the carbon element in the lanthanum dioxide carbonate catalyst comes from carbon dioxide in the CO₂-containing atmosphere.

According to the present invention, preferably, the lanthanum dioxide carbonate catalyst having a specific structure obtained by the specific preparation method of the present invention, when the lanthanum dioxide carbonate catalyst of the present invention is subjected to XRD measurement, a clear characteristic peak of the hexagonal crystalline phase can be seen in its XRD spectrum, and no characteristic peak of the doping element in the form of metal salts appears. For the purpose of the present invention, the "no characteristic peak of..." means that there is no measurable characteristic peak in the XRD spectrum. Without being limited to any known theory, it is believed that this specific structure is conducive to the catalytic activity of the lanthanum dioxide carbonate catalyst of the present invention.

In another aspect, the present invention provides a lanthanum dioxide carbonate catalyst prepared by the method described above.

In another aspect, the present invention provides a use of the above-mentioned lanthanum dioxide carbonate catalyst in the methane oxidative coupling reaction for producing C2 and higher hydrocarbons.

In another aspect, the present invention provides a method for preparing C2 or higher hydrocarbons from methane, the method comprising: in the presence of oxygen and under the conditions of methane oxidative coupling reaction, contacting methane with the above-mentioned lanthanum dioxide carbonate catalyst for reaction;
alternatively, the lanthanum dioxide carbonate catalyst is prepared according to the method of the present invention, and then methane is contacted with the obtained lanthanum dioxide carbonate catalyst in the presence of oxygen and under the conditions of methane oxidative coupling reaction.

According to the present invention, preferably, the molar ratio of the methane to the oxygen is 2:1-9:1.

According to the present invention, preferably, the temperature of the contact reaction is 500-650°C.

According to the present invention, preferably, the space velocity of methane is 5000 mL/(g·h)-200000 mL/(g·h).

### Example

The present invention will be described in detail below by way of examples, but the present invention is not limited to these examples. In the following examples:
TEM imaging was performed by using a JEOL 2100F FEG TEM with a Schottky field emission source. The accelerating voltage was 200 kV. The images obtained were selected as representatives from imaging of at least six different grid areas.

The length and diameter of the fibrous catalyst were measured by using the ruler in the software provided with the transmission electron microscope image.

The analysis of the reaction product components was carried out on a gas chromatograph of model 7890A purchased from Agilent corporation.

The XRD diffraction spectrum was obtained by an X-ray diffractometer (XRD) of model Empyrean manufactured by Malvern Panalytical, using Cu target generator, working tube voltage 40 kV, working tube current 40 mA, PixCel ^{3D} detector, divergence slit 1/4°, anti-scattering slit 1/2°, light bar 10 mm, Soller slit 0.04 rad, receiving slit 7.5 mm, scanning speed 0.013°/step, scanning time 30s/step, scanning range: 5°-90°, and measurement was performed in reflection mode.

The hexagonal crystalline phase content was determined by the XRD spectrum. Specifically, the bulk crystal structure information was measured by the XRD spectrum, and the diffraction peak area of the hexagonal crystalline lanthanum dioxide carbonate and the crystal structure containing a doping element and all the peak areas were used to calculate the ratio thereof, i.e. the weight percentage of the hexagonal crystalline lanthanum dioxide carbonate and the hexagonal crystalline lanthanum dioxide carbonate containing a doping element in the product.

The contents of lanthanum and doping element R in the catalyst were measured by iCAP TQ ICP-MS purchased from Thermo Scientific corporation.

The pore structure of the catalyst was characterized by BET analysis using an automatic adsorption instrument ASAP2420M purchased from MICROMERITICS, USA.

The calculation method of methane conversion rate is as follows: methane conversion rate = amount of methane consumed in the reaction/initial amount of methane × 100%.

The calculation method of ethylene selectivity is as follows: ethylene selectivity = amount of methane consumed to produce ethylene/total methane consumption × 100%.

The calculation method of ethane selectivity is as follows: ethane selectivity = amount of methane consumed to produce ethane/total methane consumption × 100%.

The calculation method of C2 hydrocarbons yield is as follows: C2 hydrocarbons yield = methane conversion rate × (ethane selectivity + ethylene selectivity).

### Example 1

(1) 5g of lanthanum nitrate hexahydrate was dissolved in 210g of deionized water, and stirred at 25°C and a rotation speed of 900rpm for 30min; then sodium hydroxide solution was added dropwise into the lanthanum nitrate aqueous solution (the concentration of the sodium hydroxide solution was 10wt%, the addition rate of the alkali solution was 7mL/min per kilogram of the solution of lanthanum source, and the utilization amount of the alkali solution was such that the final pH value of the mixed system of the alkali solution and the solution of lanthanum source was 11.6), and stirred it at 25°Cand a rotation speed of 9000rpm for 10min after the drop-adding was completed. Then, the temperature was raised to 90°C, the condensation reflux was maintained and the stirring was continued for 12h. After the solution was cooled to room temperature, the solid material was separated by centrifugation at 1000rpm by a centrifuge, and washed with deionized water until the pH value of the washing liquid was neutral. The obtained solid was sequentially subjected to a first drying and a second drying. The first drying conditions included: a temperature of 80°C and a time of 12h; the second drying conditions included: a relative pressure of 60kPa, a temperature of 140°C and a time of 5h.
   The saturated water absorption amount of the lanthanum hydroxide after the second drying was determined as follows: under nitrogen protection, 1g of the lanthanum hydroxide after the second drying was taken and placed in a round-bottom flask, deionized water was added dropwise to the round-bottom flask by using a syringe, and was mixed with the lanthanum hydroxide by using a vortex mixer until the lanthanum hydroxide did not absorb further water significantly, and the drop-addition of deionized water was stopped, and the addition amount of deionized water was recorded at this time. The saturated water absorption amount of lanthanum hydroxide was determined to be 2mL/g.
(2) 0.08 g of strontium nitrate was dissolved in 1.5 mL of deionized water to obtain a strontium nitrate aqueous solution. Under nitrogen protection, 0.8 g of the lanthanum hydroxide after the second drying was weighed and placed in a round-bottom flask. Then, at 25°C, the strontium nitrate solution was added dropwise into the round-bottom flask by using a syringe (the addition rate of the solution containing a compound of doping element was 1 mL/min per gram of the lanthanum hydroxide), and was fully mixed by a vortex mixer for 5 h. Then, the obtained mixture was dried at 80°C for 12h.
(3) A lanthanum dioxide carbonate catalyst was prepared by calcining at 500°C for 2h in an air atmosphere.

The molar ratio of lanthanum element to strontium element in the catalyst was 1:0.091.

BET analysis shows that the lanthanum dioxide carbonate catalyst has a specific surface area of 52 m²/g, a pore volume of 0.31 cm³/g, and an average pore diameter of 10. 1 nm.

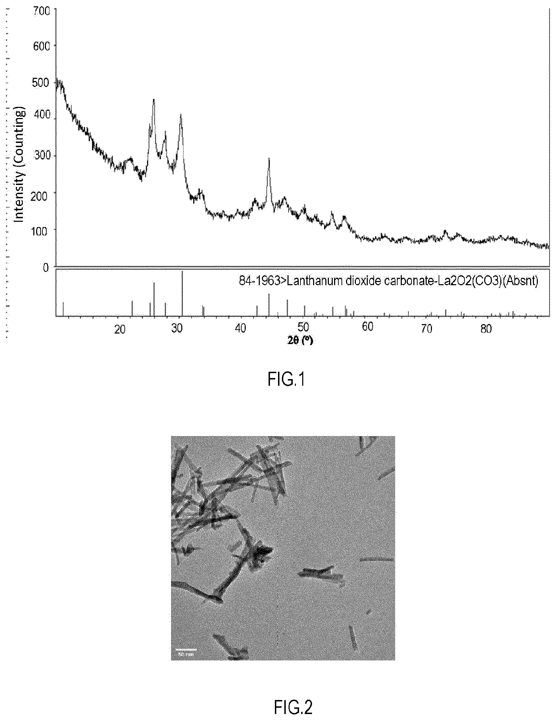

FIG.1 is an XRD spectrum of the lanthanum dioxide carbonate catalyst prepared in Example 1, wherein the abscissa is 2θ and the ordinate is intensity. Compared with the PXRD database (Bruker Diffrac. Eva, version 4.2.1), it can be seen that the catalyst prepared by this method has characteristic peaks of a hexagonal crystalline phase, and because it is nanoscale, the crystallinity is lower. It can also be seen that only the characteristic peaks of the hexagonal crystalline phase are seen in the XRD spectrum, and no characteristic peaks of the doping elements in the form of metal salts appear. The total content of hexagonal crystalline lanthanum dioxide carbonate and hexagonal crystalline lanthanum dioxide carbonate containing a doping element in the product is 98.0wt% as determined by the XRD spectrum.

FIG.2 is a transmission scanning electron microscope image of the lanthanum dioxide carbonate catalyst prepared in Example 1. It can be seen from FIG.2 that the lanthanum dioxide carbonate catalyst has a fibrous nanostructure, and it is calculated to have an average diameter of 13 nm and an average length-to-diameter ratio of 40:1.

### Example 2

(1) 5.15g of lanthanum nitrate hexahydrate was dissolved in 155g of deionized water and stirred at 25°C and a rotation speed of 900rpm for 30min, then sodium hydroxide solution was added dropwise into the lanthanum nitrate aqueous solution (the concentration of sodium hydroxide solution was 10wt%, and the addition rate of alkali solution was 5mL/min per kilogram of the solution of lanthanum source, and the utilization amount of alkali solution was such that the final pH value of the mixed system of alkali and lanthanum source was 12.2), and after drop-adding was completed, the stirring was continued at 25°Cand a rotation speed of 9000rpm for 10min, and then the temperature was raised to 100°C, condensation reflux was maintained and the stirring was continued for 15 h. After the solution was cooled down to room temperature, the solid material was centrifuged by centrifugation at 9000 rpm by a centrifuge, and washed with deionized water until the pH value of the washing liquid was neutral, and the obtained solid was subjected to a first drying and a second drying sequentially, and the first drying conditions included: a temperature of 80°C and a time of 15 h; the second drying conditions included: a relative pressure of 50kPa, a temperature of 150°C, and a time of 5 h.
   The saturated water absorption amount of the lanthanum hydroxide after the second drying was determined as follows: 1g of the lanthanum hydroxide after the second drying was taken and placed into a round-bottomed flask under the protection of nitrogen, and deionized water was added dropwise into the round-bottomed flask by using a syringe, and was mixed with the lanthanum hydroxide by using a vortex mixer until the lanthanum hydroxide did not have obvious further water absorption, stopped adding dropwise the deionized water, and the addition amount of the deionized water was recorded at this time. The saturated water absorption amount of lanthanum hydroxide was determined to be 2.1 mL/g.
(2) A strontium nitrate aqueous solution was obtained by dissolving 0.04 g of strontium nitrate in 1.68 mL of deionized water. Under nitrogen protection, 0.9 g of the lanthanum hydroxide after the second drying was weighed and placed in a round-bottomed flask, and then the strontium nitrate aqueous solution was added dropwise to the round-bottomed flask by using a syringe at 30°C (the addition rate of the solution of the compound containing a doping element was 4 mL/min per gram of the said lanthanum hydroxide), and a thorough mixing thereof was conducted by a vortex mixer, and the mixing time was 2h. The obtained mixture was dried at 80°C for 13 h.
(3) The lanthanum dioxide carbonate catalyst was produced by calcining at 500°C for 2h in an air atmosphere.

The molar ratio of lanthanum element to strontium element in the catalyst was 1:0.046.

BET analysis shows that the lanthanum dioxide carbonate catalyst has a specific surface area of 56 m²/g, a pore volume of 0.4 cm³/g, and an average pore size of 13.4 nm.

Only characteristic peaks of hexagonal crystalline phases are seen in the XRD spectrum (similar to Example 1, not shown again), and there are no characteristic peaks of said doping element in the form of metal salt. The total content of the hexagonal crystalline lanthanum dioxide carbonate and the hexagonal crystalline lanthanum dioxide carbonate containing a doping element in the product is determined by XRD spectroscopy to be 98.6wt%.

From the observation of the transmission scanning electron micrograph (similar to Example 1 and not shown again), it can be seen that the lanthanum dioxide carbonate catalyst has a fibrous nanostructure and it is calculated to have an average diameter of 14 nm and an average length-to-diameter ratio of 35:1.

### Example 3

(1) 10 g of lanthanum nitrate hexahydrate was dissolved in 155 g of deionized water, stirred at 25°C and at a rotation speed of 900 rpm for 30 min, then sodium hydroxide solution was added dropwise into the lanthanum nitrate aqueous solution (the concentration of the sodium hydroxide solution was 10wt%, the addition rate of the alkali solution was 7 mL/min per kilogram of the solution of lanthanum source, and the utilization amount of the alkali solution was such that the final pH value of the mixed system of the alkali solution and the solution of lanthanum source was 12), and after the drop-addition is completed, stirring was continued at 25°C and at a rotation speed of 9000 rpm for 10 min, and then the temperature was increased to 100°C with continuous stirring at condensation reflux for 15h. After the solution was cooled to room temperature, the solid material was separated by centrifugation at 900 rpm in a centrifuge, and washed with deionized water until the pH value of the washing liquid was neutral. The obtained solid was sequentially subjected to a first drying and a second drying. The first drying conditions included: a temperature of 90°C and a time of 20 h; the second drying conditions included: a relative pressure of 50kPa, a temperature of 160°C and a time of 3 h.
   The saturated water absorption amount of lanthanum hydroxide after the second drying was determined as follows: under nitrogen protection,1g of lanthanum hydroxide after the second drying was taken and placed in a round-bottom flask, deionized water was added dropwise to the round-bottom flask by using a syringe, and was mixed with lanthanum hydroxide by using a vortex mixer until lanthanum hydroxide did not absorb further water significantly, and the drop-adding the deionized water was stopped , and the addition amount of deionized water was recorded at this time. The saturated water absorption amount of lanthanum hydroxide was determined to be 2.4 mL/g.
(2) 0.13 g of strontium nitrate was dissolved in 1.92 mL of deionized water to obtain a strontium nitrate aqueous solution. Under nitrogen protection, 0.9 g of the lanthanum hydroxide after the second drying was weighed and placed in a round-bottom flask. Then, at 40°C, the strontium nitrate aqueous solution was added dropwise into the round-bottom flask by using a syringe (the addition rate of the solution containing a compound of doping element was 3 mL/min per gram of the lanthanum hydroxide), and was fully mixed by a vortex mixer for 2 h. Then, the obtained mixture was dried at 80°C for 10 h.
(3) A lanthanum dioxide carbonate catalyst was prepared by calcining at 500°C for 2h in an air atmosphere.

The molar ratio of lanthanum element to strontium element in the catalyst was 1:0.148.

BET analysis shows that the lanthanum dioxide carbonate catalyst has a specific surface area of 51 m²/g, a pore volume of 0.3 cm³/g, and an average pore diameter of 10.5 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 99.1wt% as determined by the XRD spectrum.

From the transmission scanning electron microscopy image (similar to Example 1, not shown again), it can be seen that the lanthanum dioxide carbonate catalyst has a fibrous nanostructure, and it is calculated to have an average diameter of 18 nm and an average length-to-diameter ratio of 20:1.

### Example 4

(1) 15 g of lanthanum nitrate hexahydrate was dissolved in 155 g of deionized water, and was stirred at 25°C and at a rotation speed of 9000 rpm for 30 min. Then, a sodium hydroxide solution was added dropwise to the lanthanum nitrate aqueous solution (the concentration of the sodium hydroxide solution was 10wt%, calculated as the compound of the Group IA metal, and the addition rate of the alkali solution was 10 mL/min per kilogram of the solution of lanthanum source; the utilization amount of the alkali solution was such that the final pH value of the mixed system of the alkali solution and the solution of lanthanum source was 10). After the drop-adding was completed, the stirring at 25°C and at a rotation speed of 900 rpm was continued for 10 min. Then the temperature was raised to 100°C and the condensation reflux was maintained with continuous stirring for 15 h. After the solution was cooled to room temperature, the solid material was separated by centrifugation at 9000 rpm in a centrifuge and washed with deionized water until the pH value of the washing liquid was neutral. The obtained solid was sequentially subjected to a first drying and a second drying. The first drying conditions included: a temperature of 80°C and a time of 15 h; the second drying conditions included: a relative pressure of 40kPa, a temperature of 140°C, and a time of 5 h.
   The saturated water absorption amount of the lanthanum hydroxide after the second drying was determined: under nitrogen protection, 1g of the lanthanum hydroxide after the second drying was taken and placed in a round-bottom flask, deionized water was added dropwise to the round-bottom flask by using a syringe, and was mixed with the lanthanum hydroxide by using a vortex mixer until the lanthanum hydroxide did not absorb further water significantly, and the drop-adding the deionized water was stopped , and the addition amount of deionized water was recorded at this time. The saturated water absorption amount of lanthanum hydroxide was determined to be 1.9 mL/g.
(2) 0.24 g of strontium nitrate was dissolved in 1.52 mL of deionized water to obtain a strontium nitrate aqueous solution. Under nitrogen protection, 0.9 g of the lanthanum hydroxide after the second drying was weighed and placed in a round-bottom flask. Then, at 35°C, the strontium nitrate aqueous solution was added dropwise into the round-bottom flask by using a syringe (per gram of the lanthanum hydroxide, the addition rate of the solution containing a compound of doping element was 1.5 mL/min), and fully mixed by a vortex mixer, the mixing time was 3 hours. Then, the obtained mixture was dried at 80°C for 15 h.
(3) A lanthanum dioxide carbonate catalyst was prepared by calcining at 500°C for 2h in an air atmosphere.

The molar ratio of lanthanum element to strontium element in the catalyst was 1:0.146.

BET analysis shows that the lanthanum dioxide carbonate catalyst has a specific surface area of 50 m²/g, a pore volume of 0.26 cm³/g, and an average pore diameter of 11.6 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 99.4wt% as determined by the XRD spectrum.

From the transmission scanning electron microscopy image (similar to Example 1, not shown again), it can be seen that the lanthanum dioxide carbonate catalyst has a fibrous nanostructure, and it is calculated to have an average diameter of 16 nm and an average length-to-diameter ratio of 15:1.

### Example 5

Lanthanum dioxide carbonate was prepared according to the method of Example 1, except that strontium nitrate was replaced by an equimolar amount of barium nitrate.

BET analysis shows that the lanthanum dioxide carbonate catalyst has a specific surface area of 49 m²/g, a pore volume of 0.29 cm³/g, and an average pore diameter of 12.6 nm.

From the transmission scanning electron microscopy image (similar to Example 1, not shown again), it can be seen that the lanthanum dioxide carbonate catalyst has a fibrous nanostructure, and it is calculated to have an average diameter of 22 nm and an average length-to-diameter ratio of 19:1.

### Example 6

Lanthanum dioxide carbonate was prepared according to the method of Example 1, except that in step (1), the second drying step was not included.

BET analysis shows that the lanthanum dioxide carbonate catalyst has a specific surface area of 68 m²/g, a pore volume of 0.39 cm³/g, and an average pore diameter of 8.5 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 98.7wt% as determined by the XRD spectrum.

From the transmission scanning electron microscopy, it can be seen that the lanthanum dioxide carbonate catalyst has a fibrous nanostructure, and it is calculated to have an average diameter of 21 nm and an average length-to-diameter ratio of 16:1.

### Example 7

Lanthanum dioxide carbonate was prepared according to the method of Example 1, except that strontium nitrate was replaced by an equimolar amount of zinc nitrate.

BET analysis shows that the lanthanum dioxide carbonate catalyst has a specific surface area of 42 m²/g, a pore volume of 0.21 cm³/g, and an average pore diameter of 9.8 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 98.2wt% as determined by the XRD spectrum.

From the transmission scanning electron microscopy image (similar to Example 1, not shown again), it can be seen that the lanthanum dioxide carbonate catalyst has a fibrous nanostructure, and it is calculated to have an average diameter of 23 nm and an average length-to-diameter ratio of 18:1.

### Example 8

Lanthanum dioxide carbonate was prepared according to the method of Example 1, except that strontium nitrate was replaced by an equimolar amount of magnesium nitrate.

BET analysis shows that the lanthanum dioxide carbonate catalyst has a specific surface area of 39 m²/g, a pore volume of 0.46 cm³/g, and an average pore diameter of 15.2 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 98.9wt% as determined by the XRD spectrum.

From the transmission scanning electron microscopy image (similar to Example 1, not shown again), it can be seen that the lanthanum dioxide carbonate catalyst has a fibrous nanostructure, and it is calculated to have an average diameter of 20 nm and an average length-to-diameter ratio of 16:1.

### Example 9

Lanthanum dioxide carbonate was prepared according to the method of Example 1, except that in step (2), the lanthanum hydroxide after the second drying was added to the strontium nitrate aqueous solution, was mixed and stirred for 5 h, and then the obtained mixture was dried at 80°C for 12 hours.

BET analysis shows that the lanthanum dioxide carbonate catalyst has a specific surface area of 39 m²/g, a pore volume of 0.31 cm³/g, and an average pore diameter of 12.8 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 99.3wt% as determined by the XRD spectrum.

From the transmission scanning electron microscopy image (similar to Example 1, not shown again), it can be seen that the lanthanum dioxide carbonate catalyst has a fibrous nanostructure, and it is calculated to have an average diameter of 16 nm and an average length-to-diameter ratio of 19:1.

### Example 10

Lanthanum dioxide carbonate was prepared according to the method of Example 1, except that the addition rate of the solution containing a compound of doping element was 20 mL/min per gram of the lanthanum hydroxide.

BET analysis shows that the lanthanum dioxide carbonate catalyst has a specific surface area of 44 m²/g, a pore volume of 0.28 cm³/g, and an average pore diameter of 11.2 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 99.5wt% as determined by the XRD spectrum.

It can be seen from the transmission scanning electron microscopy image (similar to Example 1, not shown again) that the lanthanum dioxide carbonate catalyst has a fibrous nanostructure, and it is calculated to have an average diameter of 20 nm and an average length-to-diameter ratio of 18.6:1.

### Example 11

Lanthanum dioxide carbonate was prepared according to the method of Example 1, except that in step (2), the amount of deionized water used was 5mL.

BET analysis shows that the lanthanum dioxide carbonate catalyst has a specific surface area of 38 m²/g, a pore volume of 0.2 cm³/g, and an average pore diameter of 13.3 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 99.6wt% as determined by the XRD spectrum.

From the transmission scanning electron microscopy image (similar to Example 1, not shown again), it can be seen that the lanthanum dioxide carbonate catalyst has a fibrous nanostructure, and it is calculated to have an average diameter of 21 nm and an average length-to-diameter ratio of 17:1.

### Example 12

(1) 15 g of lanthanum nitrate hexahydrate was dissolved in 155 g of deionized water, and was stirred at 25°C and at a rotation speed of 900 rpm for 30 min. Then, a sodium hydroxide solution was added dropwise to the lanthanum nitrate aqueous solution (the concentration of the sodium hydroxide solution was 10wt%, calculated as the compound of the Group IA metal, the addition rate of the alkali solution was 10 mL/min per kilogram of the solution of lanthanum source; and the utilization amount of the alkali solution was such that the final pH value of the mixed system of the alkali solution and the solution of lanthanum source was 10). After the drop-adding was completed, the mixture was stirred at 25°C and at a rotation speed of 9000 rpm for 10 min. Then the temperature was raised to 100°C and the condensation reflux was maintained with continuous stirring for 15 h. After the solution was cooled to room temperature, the solid material was separated by centrifugation at 9000 rpm in a centrifuge and washed with deionized water until the pH value of the washing liquid was neutral. The obtained solid was sequentially subjected to a first drying and a second drying. The first drying conditions included: a temperature of 80°C and a time of 15 h; the second drying conditions included: a relative pressure of 40kPa, a temperature of 140°C, and a time of 5 h.
   the saturated water absorption amount of the lanthanum hydroxide after the second drying was determined: under nitrogen protection, 1g of the lanthanum hydroxide after the second drying was taken and placed in a round-bottom flask, deionized water was added dropwise to the round-bottom flask by using a syringe, and was mixed with the lanthanum hydroxide by using a vortex mixer until the lanthanum hydroxide did not absorb further water significantly, and the drop-adding the deionized water was stopped , and the addition amount of deionized water was recorded at this time. The saturated water absorption amount of lanthanum hydroxide was determined to be 1.9 mL/g.
(2) 0.24 g of ferric nitrate was dissolved in 1.52 mL of deionized water to obtain a strontium nitrate aqueous solution. Under nitrogen protection, 0.9 g of the lanthanum hydroxide after the second drying was weighed and placed in a round-bottom flask. Then, at 35°C, the ferric nitrate aqueous solution was added dropwise into the round-bottom flask by using a syringe (the addition rate of the solution containing a compound of doping element was 1.5 mL/min per gram of the lanthanum hydroxide), and fully mixed by a vortex mixer, the mixing time was 3 hours. Then, the obtained mixture was dried at 80°C for 15 h.
(3) A lanthanum dioxide carbonate catalyst was prepared by calcining at 500°C for 2h in an air atmosphere.

The molar ratio of lanthanum element to the ferric element in the catalyst was 1:0.13.

BET analysis shows that the lanthanum dioxide carbonate catalyst has a specific surface area of 48 m²/g, a pore volume of 0.36 cm³/g, and an average pore diameter of 13.8 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 98.1wt% as determined by the XRD spectrum.

It can be seen from the transmission scanning electron microscopy image (similar to Example 1, not shown again) that the lanthanum dioxide carbonate catalyst has a fibrous nanostructure, and it is calculated to have an average diameter of 20 nm and an average length-to-diameter ratio of 20:1.

### Example 13

(1) 5g of lanthanum nitrate hexahydrate was dissolved in 210g of deionized water, and stirred at 25°C and a rotation speed of 900rpm for 30min; then sodium hydroxide solution was added dropwise into the lanthanum nitrate aqueous solution (the concentration of the sodium hydroxide solution was 10wt%, the addition rate of the alkali solution was 7mL/min per kilogram of the solution of lanthanum source, and the utilization amount of the alkali solution was such that the final pH value of the mixed system of the alkali solution and the solution of lanthanum source was 11.6 ), and stirring at 25°C and 9000rpm was continued for 10min after the drop-adding was completed. Then, the temperature was raised to 90°C, and the condensation reflux was maintained with stirring for 12h. After the solution was cooled to room temperature, the solid material was separated by centrifugation at 1000rpm by a centrifuge, and washed with deionized water until the pH value of the washing liquid was neutral. The obtained solid was sequentially subjected to a first drying and a second drying. The first drying conditions included: a temperature of 80°C and a time of 12h; the second drying conditions included: a relative pressure of 60kPa, a temperature of 140°C and a time of 5h.
   The saturated water absorption amount of the lanthanum hydroxide after the second drying was determined: under nitrogen protection, 1g of the lanthanum hydroxide after the second drying was taken and placed in a round-bottom flask, deionized water was added dropwise to the round-bottom flask by using a syringe, and was mixed with the lanthanum hydroxide by using a vortex mixer until the lanthanum hydroxide did not absorb further water significantly, and the drop-adding the deionized water was stopped , and the addition amount of deionized water was recorded at this time. The saturated water absorption amount of lanthanum hydroxide was determined to be 2mL/g.
(2) 0.06 g of calcium nitrate was dissolved in 1.5 mL of deionized water to obtain a calcium nitrate aqueous solution. Under nitrogen protection, 1.0 g of the lanthanum hydroxide after the second drying was weighed and placed in a round-bottom flask. Then, at 25°C, the calcium nitrate aqueous solution was added dropwise into the round-bottom flask by using a syringe (the addition rate of the solution of the compound containing doping elemental calcium was 1 mL/min per gram of the lanthanum hydroxide), and was fully mixed by a vortex mixer for 5 h. Then, the obtained mixture was dried at 80°C for 12 hours.
(3) A lanthanum dioxide carbonate catalyst was prepared by calcining at 500°C for 2h in an air atmosphere.

The molar ratio of lanthanum element to calcium element in the catalyst was 1:0.11.

BET analysis shows that the lanthanum dioxide carbonate catalyst has a specific surface area of 49 m²/g, a pore volume of 0.3 cm³/g, and an average pore diameter of 10.2 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 99.3wt% as determined by the XRD spectrum.

It can be seen from the transmission scanning electron microscopy image (similar to Example 1, not shown again) that the lanthanum dioxide carbonate catalyst has a fibrous nanostructure, and it is calculated to have an average diameter of 18 nm and an average length-to-diameter ratio of 20:1.

### Comparative Example 1

(1) 5g of lanthanum nitrate hexahydrate was dissolved in 210g of deionized water, stirred at 25°C and a rotation speed of 900rpm for 30min, then sodium hydroxide solution was added dropwise into the lanthanum nitrate aqueous solution (the concentration of the sodium hydroxide solution was 10wt%, the addition rate of the alkali solution was 7mL/min per kilogram of the solution of lanthanum source, and the utilization amount of the alkali solution was such that the final pH value of the mixed system of the alkali solution and the solution of lanthanum source was 11.6), and after the drop-adding was completed, stirring was continued at 25°Cand a rotation speed of 9000rpm for 10min. Then, temperature was raised to 90°C and condensation reflux was maintained with stirring for 12h. After the solution was cooled to room temperature, the solid material by centrifugation was separated at 10000rpm in a centrifuge, and was washed with deionized water until the pH value of the washing liquid was neutral. The obtained solid was sequentially subjected to a first drying and a second drying. The first drying conditions included: a temperature of 80°C and a time of 12h; the second drying conditions included: a relative pressure of 60kPa, a temperature of 140°C and a time of 5h. A lanthanum dioxide carbonate catalyst was prepared by calcining at 500°C for 2h in air atmosphere.

BET analysis shows that the lanthanum dioxide carbonate catalyst has a specific surface area of 68 m²/g, a pore volume of 6 cm³/g, and an average pore diameter of 42 nm.

Only the characteristic peaks of the hexagonal crystalline phase are seen in the XRD spectrum (similar to Example 1, not shown again), and no characteristic peaks of the doping element in the form of metal salts appear. The total content of hexagonal crystalline lanthanum dioxide carbonate in the product was 98.2wt% as determined by the XRD spectrum.

From the transmission scanning electron microscopy image (similar to Example 1, not shown again), it can be seen that the lanthanum dioxide carbonate catalyst has a fibrous nanostructure, and it is calculated to have an average diameter of 12 nm and an average length-to-diameter ratio of 15:1.

### Comparative Example 2

(1) Lanthanum hydroxide was prepared according to the method of step (1) of Example 1;
(2) 0.08 g of strontium nitrate was calcined at 500°C for 2h in an air atmosphere;
(3) 0.8 g of lanthanum hydroxide was calcined at 500°C for 2h in an air atmosphere;
(4) then, the product obtained in step (2) and the product obtained in step (3) were mixed evenly to obtain a catalyst.

BET analysis shows that the lanthanum dioxide carbonate catalyst has a specific surface area of 40 m²/g, a pore volume of 2.3 cm³/g, and an average pore diameter of 14.2 nm.

Only the characteristic peaks of the hexagonal crystalline phase are seen in the XRD spectrum (similar to Example 1, not shown again), and no characteristic peaks of the doping element in the form of metal salts appear. The total content of the hexagonal crystalline lanthanum dioxide carbonate and the hexagonal crystalline lanthanum dioxide carbonate containing a doping element in the product was 82.3wt% as determined by the XRD spectrum.

### Comparative Example 3

(1) 5g of lanthanum nitrate hexahydrate was dissolved in 210g of deionized water, stirred at 25°C and a rotation speed of 900rpm for 30min, then sodium hydroxide solution was added dropwise into the lanthanum nitrate aqueous solution (the concentration of the sodium hydroxide solution was 10wt%, the addition rate of the alkali solution was 7mL/min per kilogram of the solution of lanthanum source, and the utilization amount of the alkali solution was such that the final pH value of the mixed system of the alkali solution and the solution of lanthanum source was 11.6), and stirring at 25°C and a rotation speed of 9000rpm was continued for 10min after the drop-adding was completed. Then, the temperature was raised to 90°C and condensation reflux was kept with stirring for 12h. After the solution was cooled to room temperature, the solid material was separated by centrifugation at 10000rpm in a centrifuge, and was washed with deionized water until the pH value of the washing liquid was neutral. The obtained solid was sequentially subjected to a first drying and a second drying. The first drying conditions included: a temperature of 80°C and a time of 12h; the second drying conditions included: a relative pressure of 60kPa, a temperature of 140°C and a time of 5h.
   The saturated water absorption amount of the lanthanum hydroxide after the second drying was determined: under nitrogen protection, 1g of the lanthanum hydroxide after the second drying was taken and placed in a round-bottom flask, deionized water was added dropwise to the round-bottom flask by using a syringe, and was mixed with the lanthanum hydroxide by using a vortex mixer until the lanthanum hydroxide did not absorb further water significantly, and adding deionized water was stopped; and the addition amount of deionized water was recorded at this time. The saturated water absorption amount of lanthanum hydroxide was determined to be 2mL/g.
(2) 0.08 g of strontium nitrate was dissolved in 1.5 mL of deionized water to obtain a strontium nitrate aqueous solution. Under nitrogen protection, 0.8 g of the lanthanum hydroxide after the second drying was weighed, and then the lanthanum hydroxide solid was added directly to the strontium nitrate solution at 25°C. Then the obtained mixture was dried at 80°C for 12 h.
(3) A lanthanum dioxide carbonate catalyst was prepared by calcining at 500°C for 2h in an air atmosphere.

### Comparative Example 4

0.26 g of barium nitrate was dissolved in 10 mL of deionized water and was stirred to dissolve it completely. 2g of the prepared hexagonal crystalline lanthanum dioxide carbonate was weighed, the barium nitrate aqueous solution was dropwise added into the hexagonal crystalline lanthanum dioxide carbonate, was stirred to mix it evenly and placed in an 80°C oven for 24 h, then it is transferred to a muffle furnace, heated to 500°C at 2°C/min, and maintained for 2h to prepare Ba/La₂O₂CO₃.

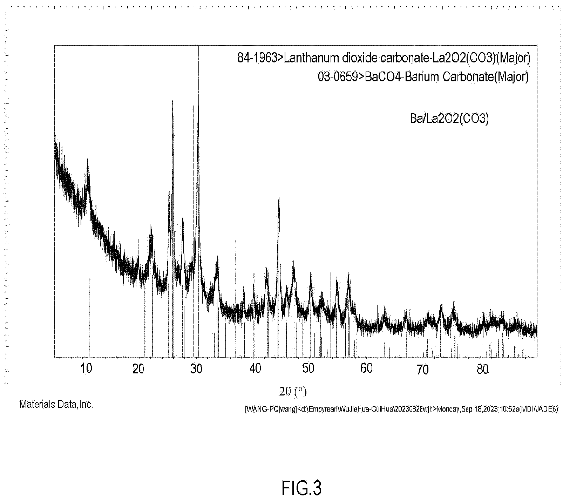

FIG.3 is an XRD spectrum of the lanthanum dioxide carbonate catalyst prepared in Comparative Example 4, from which it can be seen that: XRD results show that in addition to the peaks of lanthanum dioxide carbonate, a characteristic peak of BaCO₄ is also obtained in the sample.

### Comparative Example 5

0.3 g of nickel nitrate hexahydrate was dissolved in 10 mL of deionized water and stirred to dissolve it completely. 2g of the prepared hexagonal crystalline lanthanum dioxide carbonate was weighed, the nickel nitrate aqueous solution was added dropwise into the hexagonal crystalline lanthanum dioxide carbonate, and was stirred to mix them evenly, placed in an oven at 80°C for 24 h, and then transferred to a muffle furnace; the temperature was raised to 500°C at 2°C/min, and maintained for 2h to prepare Ni/La₂O₂CO₃.

### Test Example 1

The catalysts prepared in the above examples and comparative examples were tableted and sieved through 40-60 meshes, and then 0.1g was taken and loaded into an Inconel fixed-bed reactors. Under normal pressure, methane and oxygen (the molar ratio of methane to oxygen was 3:1) were introduced to react. Other reaction conditions and results were shown in Table 1.

**Table 1**

| | Reaction temperature | Methane space velocity | Methane conversion rate | C2 hydrocarbons selectivity | Yield of C2 hydrocarbons |
|---|---|---|---|---|---|
| | °C | mL/g·h | % | % | % |
| Example 1 | 500 | 40000 | 30.8 | 48.7 | 15.00 |
| | 550 | 40000 | 31.8 | 52.7 | 16.76 |
| | 600 | 40000 | 33.5 | 52.3 | 17.52 |
| Example 2 | 500 | 40000 | 26.1 | 54.7 | 14.28 |
| | 550 | 40000 | 29.8 | 47.8 | 14.24 |
| | 600 | 40000 | 30.7 | 48.9 | 15.01 |
| Example 3 | 500 | 40000 | 31.5 | 44.6 | 14.05 |
| | 550 | 40000 | 32.1 | 46.3 | 14.86 |
| | 600 | 40000 | 33.5 | 52.3 | 17.52 |
| Example 4 | 500 | 80000 | 27.3 | 56.2 | 15.34 |
| | 550 | 80000 | 28.4 | 57.6 | 16.36 |
| | 600 | 80000 | 29.8 | 60.5 | 18.03 |
| Example 5 | 500 | 50000 | 26.9 | 57.6 | 15.49 |
| | 550 | 50000 | 30.9 | 53.1 | 16.41 |
| | 600 | 50000 | 31.3 | 51.1 | 15.99 |
| Example 6 | 500 | 50000 | 32.4 | 39.6 | 12.83 |
| | 550 | 50000 | 33.7 | 41.1 | 13.85 |
| | 600 | 50000 | 34.6 | 42.3 | 14.64 |
| Example 7 | 500 | 50000 | 24.8 | 47.4 | 11.76 |
| | 550 | 50000 | 26.7 | 46.7 | 12.47 |
| | 600 | 50000 | 31.7 | 43.7 | 13.85 |
| Example 8 | 500 | 50000 | 27.5 | 56.3 | 15.48 |
| | 550 | 50000 | 28.3 | 47.7 | 13.50 |
| | 600 | 50000 | 31 | 50.3 | 15.59 |
| Example 9 | 500 | 50000 | 23.6 | 45.6 | 10.76 |
| | 550 | 50000 | 27.9 | 46.2 | 12.89 |
| | 600 | 50000 | 28.2 | 47.1 | 13.28 |
| Example 10 | 500 | 50000 | 26.3 | 43.1 | 11.34 |
| | 550 | 50000 | 28.3 | 46.8 | 13.24 |
| | 600 | 50000 | 28.6 | 46.9 | 13.41 |
| Embodiment 11 | 500 | 50000 | 25.6 | 43.1 | 11.03 |
| | 550 | 50000 | 26.6 | 45.9 | 12.21 |
| | 600 | 50000 | 27.6 | 46.9 | 12.94 |
| Example 12 | 500 | 50000 | 25.4 | 48 | 12.19 |
| | 550 | 50000 | 26.5 | 48.4 | 12.83 |
| | 600 | 50000 | 27.4 | 47.9 | 13.12 |
| Example 13 | 500 | 40000 | 27.4 | 56.2 | 15.3 |
| | 550 | 40000 | 32.8 | 45.4 | 14.9 |
| | 600 | 40000 | 29.7 | 53.5 | 15.9 |
| Comparative Example 1 | 500 | 50000 | 21 | 43.4 | 9.11 |
| | 550 | 50000 | 23.6 | 43.1 | 10.17 |
| | 600 | 50000 | 24.6 | 44.2 | 10.87 |
| Comparative Example 2 | 500 | 50000 | 18 | 43.6 | 7.85 |
| | 550 | 50000 | 20 | 44.2 | 8.84 |
| | 600 | 50000 | 20 | 44.3 | 8.86 |
| Comparative Example 3 | 500 | 50000 | 19.4 | 44.3 | 8.59 |
| | 550 | 50000 | 22.1 | 44.5 | 9.83 |
| | 600 | 50000 | 22.1 | 43.5 | 9.61 |
| Comparative Example 4 | 500 | 50000 | 2.3 | 1.6 | 0.04 |
| | 550 | 50000 | 19 | 46.7 | 8.87 |
| | 600 | 50000 | 20 | 49.9 | 9.98 |
| Comparative Example 5 | 500 | 40000 | 30.2 | 10.2 | 3.1 |
| | 550 | 40000 | 33.7 | 8.0 | 2.7 |
| | 600 | 40000 | 40.0 | 3.9 | 1.6 |

It can be seen from the results in Table 1 that, compared with Comparative Example 1, the utilization of the lanthanum dioxide carbonate catalyst containing a doping element R of the present invention produces a higher yield of C2 hydrocarbons at 500-650°C. Particularly preferably, the yield of C2 hydrocarbons using the catalysts of Examples 1-5 is higher than 15% at 550-650°C.

In Comparative Example 2, the yield of C2 hydrogens produced by the catalyst obtained by directly mixing lanthanum dioxide carbonate and strontium oxide is less than 10%, while the yield of C2 hydrogens produced by the catalyst prepared by the method of Example 1 of the present invention is 16.8%. Therefore, compared with the direct mixing method of Comparative Example 2, the element doping method of the present invention can improve the yield of C2 hydrogens.

In Comparative Example 3, solid lanthanum hydroxide was added to a strontium nitrate solution instead of dropwise adding the strontium nitrate aqueous solution to the solid lanthanum hydroxide according to the present invention. The yield of C2 hydrocarbons produced by the obtained catalyst was less than 10%.

Comparative Example 4 uses a conventional impregnation method to conduct the doping, and the obtained catalyst has lower methane conversion rate, C2 hydrocarbons selectivity and yield. In particular, the performance of the catalyst obtained by this method is greatly affected by temperature, especially at 500°C, the three important indicators of conversion rate, selectivity and yield are very low.

Comparative Example 5 uses Ni instead of the specific doping element of the present invention to conduct the doping, which obviously causes the production of the target product C2 hydrocarbons to fail to achieve the desired purpose, and the selectivity and yield of C2 hydrocarbons at various temperatures are very low.

The preferred embodiments of the present invention are described in detail above, but the present invention is not limited thereto. Within the technical concept of the present invention, the technical solution of the present invention can be subjected to a variety of simple modifications, including the combinations of various technical features in any other suitable manner, and these simple modifications and combinations should also be regarded as the contents disclosed by the present invention and belong to the protection scope of the present invention.

## Claims

1. A lanthanum dioxide carbonate catalyst, **characterized in that** the catalyst comprises hexagonal crystalline lanthanum dioxide carbonate and hexagonal crystalline lanthanum dioxide carbonate containing a doping element R, and the total content of hexagonal crystalline lanthanum dioxide carbonate and hexagonal crystalline lanthanum dioxide carbonate containing a doping element R in the catalyst is not less than 98wt%, wherein the molar ratio of the lanthanum element to the doping element R is 1:0.01 to 1:0.3; and the doping element R is selected from at least one of Mg, Ca, Sr, Ba, Fe and Zn elements.

2. The lanthanum dioxide carbonate catalyst according to claim 1, wherein the doping element R is Sr and/or Ba.

3. The lanthanum dioxide carbonate catalyst according to claim 1, wherein the total content of hexagonal crystalline lanthanum dioxide carbonate and hexagonal crystalline lanthanum dioxide carbonate containing a doping element R in the lanthanum dioxide carbonate catalyst is not less than 99wt%; and/or
the molar ratio of the lanthanum element to the doping element R in the lanthanum dioxide carbonate catalyst is 1:0.03 to 1:0.2, preferably from 1:0.04 to 1:0.15.

4. The lanthanum dioxide carbonate catalyst according to any one of claims 1 to 3, wherein the lanthanum dioxide carbonate catalyst has a fibrous nanostructure; the diameter of the lanthanum dioxide carbonate catalyst is 10 nm to 30 nm, preferably 10 nm to 20 nm, and the length-to-diameter ratio is 5:1 to 50:1, preferably 15:1 to 40:1; and/or,
the lanthanum dioxide carbonate catalyst has a specific surface area of 35 m²/g-90 m²/g, preferably 50 m²/g-60 m²/g, a pore volume of 0.15 cm³/g-0.5 cm³/g, preferably 0.26 cm³/g- 0.4 cm³/g, and an average pore diameter of 8 nm-1.6 nm, preferably 10 nm-13.5 nm.

5. The lanthanum dioxide carbonate catalyst according to any one of claims 1 to 3, wherein the XRD spectrum of the lanthanum dioxide carbonate catalyst has no characteristic peak of the doping element in the form of metal salt.

6. A method for preparing the lanthanum dioxide carbonate catalyst according to any one of the preceding claims, **characterized in that** the method comprises:
(1) adding an alkali solution to a solution of lanthanum source, and then performing a solid-liquid separation and a drying to obtain solid lanthanum hydroxide; wherein the drying comprises a first drying and a second drying, wherein the first drying conditions comprise: a temperature of 70-90°C and a time of 10-20 hours, and the second drying is performed at a higher temperature and for a shorter time than the first drying;
determining the saturated water absorption amount of the lanthanum hydroxide after the second drying: under nitrogen protection, taking 1g of the lanthanum hydroxide after the second drying and placing it in a container, adding water to evenly mix it with the lanthanum hydroxide, until the lanthanum hydroxide has no obvious further water absorption, and recording the addition amount of water at this time as the saturated water absorption amount of the lanthanum hydroxide;
(2) adding a solution of a compound containing the doping element R to the solid lanthanum hydroxide obtained in step (1) so that the solution in an amount less than or equal to the saturated water absorption amount of the lanthanum hydroxide is contacted with the lanthanum hydroxide, and then performing a drying; wherein the contacting method is selected from lattice doping, surface precipitation, atomic layer deposition and single atomic layer plating;
(3) calcining the dried product of step (2) in a carbon-containing atmosphere to obtain a lanthanum dioxide carbonate catalyst.

7. The method according to claim 6, wherein the alkali in the alkali solution is a compound of a Group IA metal, and preferably the concentration of the alkali solution is 3wt%-25wt%; and/or,
the amount of the alkali solution is such that the final pH value of the mixed system of the alkali solution and the solution of lanthanum source is 10-12.5; and/or,
the lanthanum source is a water-soluble salt of lanthanum, preferably at least one of lanthanum nitrate, lanthanum chloride and lanthanum acetate, preferably, the concentration of the solution of lanthanum source is 0.01wt%-10wt%.

8. The method according to claim 6, wherein in step (1), an aging is performed before the solid-liquid separation, and the aging conditions include: a temperature of 80-100°C and a time of 10-50h; and/or,
preferably, the second drying conditions include: a relative pressure of 10kPa-91kPa, preferably 20kPa-70kPa, a temperature of 120-160°C, and a time of 2-10 h.

9. The method according to claim 6, wherein step (2) is carried out under a protective atmosphere provided by an inert gas and/or nitrogen; and/or
the concentration of the solution of a compound containing the doping element R is 0.01-0.2 g/mL; and/or,
the molar ratio of the lanthanum element to the doping element R in the lanthanum hydroxide is 1:0.01 to 1:1; and/or,
the compound containing the doping element R is selected from a compound of at least one element of Mg, Ca, Sr, Ba, Fe and Zn, preferably a compound of Sr and/or Ba; and/or,
the contact conditions include: a temperature of 15-50°C and a time of 2-5h.

10. The method according to claim 6, wherein in step (2), the addition rate of the solution of a compound containing the doping element R is 0.1 mL/min-10 mL/min, preferably 0.1 mL/min-8 mL/min, per gram of the lanthanum hydroxide; and/or, the drying conditions include: a temperature of 60-100°C and a time of 10-24 hours.

11. The method according to claim 6, wherein in step (3), the carbon-containing atmosphere is an atmosphere containing CO and/or CO₂; and/or
the calcination conditions include: a temperature of 450-550°C and a time of 2-8h.

12. The method according to claim 6, wherein the amount of the solution of a compound containing the doping element R contacted with the lanthanum hydroxide is equal to or substantially equal to the saturated water absorption amount of the lanthanum hydroxide.

13. Use of the lanthanum dioxide carbonate catalyst according to any one of claims 1 to 5 in the methane oxidative coupling reaction to prepare C2 or higher hydrocarbons.

14. A method for preparing C2 or higher hydrocarbons from methane, **characterized in that** the method comprises:
in the presence of oxygen and under the conditions of methane oxidative coupling reaction, contacting methane with the lanthanum dioxide carbonate catalyst according to any one of claims 1 to 5 for reaction; or,
preparing a lanthanum dioxide carbonate catalyst according to the method of any one of claims 6 to 12, and then contacting methane with the obtained lanthanum dioxide carbonate catalyst for reaction in the presence of oxygen and under the conditions of methane oxidative coupling reaction.

15. The method according to claim 13, wherein the molar ratio of methane to oxygen is 2:1 to 9:1; and/or,
the contact reaction temperature is 500-650°C; and/or, the methane space velocity is 5000 mL/(g·h)-200000 mL/(g·h).
